# EUROPEAN PATENT APPLICATION

(11) **EP 4 785 864 A1**
(43) Date of publication of application: **05.08.2026**
(21) Application number: 25315028.8
(22) Date of filing: 31.01.2025
(51) Int. Cl.: A61B 5/16, A61B 5/00, G02B 27/00, G02C 11/00

(54) **SYSTEM, HEAD-MOUNTABLE DEVICE AND METHOD FOR DETERMINING A VALUE OF A PHYSIOLOGICAL PARAMETER OF AN INDIVIDUAL**

(71) Applicant: ESSILOR INTERNATIONAL, 94220 Charenton-Le-Pont (FR)
(72) Inventor: Perrot, Stéphane, 94100 Saint Maur des Fosses (FR); Guegan, Julien, 94160 Saint Mande (FR); Xu, Qi, 77420 Champs sur Marne (FR); Lassalle, Astrid, 94100 Saint Maur des Fosses (FR); Thirard, Mélissa, 95220 Herblay (FR); Akouvi, Brice, 95250 Beauchamp (FR); Gilbert, Cédric, 92160 Antony (FR); Gourraud, Alexandre, 94120 Fontenay sous Bois (FR); PApin, Juliette, 93100 Montreuil (FR); Hirschauer, Noé, 94240 L'Hay-les-Roses (FR)
(74) Representative: Korakis-Ménager, Sophie

(57) **Abstract**

System, head-mountable device and method for determining a value of a physiological parameter of an individual. The system comprises a head-mountable device intended to be worn by the individual. The head-mountable device comprises a first set of at least one sensor and a second set of at least one sensor. The at least one sensor of the first set comprises at least one LED and at least one photodiode. The at least one LED is arranged, so that light emitted by the at least one LED is reflected by the face of the individual and received by the at least one photodiode. Values outputted by the at least one sensor of the first set depend on an amount of the light received by the at least one photodiode. Values outputted by the at least one sensor of the second set depend on a distance between the face of the individual and the head-mountable device. The system is configured to determine the value of the physiological parameter of the individual based on values outputted by the at least one sensor of the first set and the values outputted by the at least one sensor of the second set.

## Description

### Technical field

This disclosure relates to systems, head-mountable devices and methods for determining a value of a physiological parameter of an individual.

### Background information and prior art

To determine the value of the physiological parameter of the individual, one may use a questionnaire. Using questionnaire is however time consuming and is not an objective method for determining the value. Furthermore, if the values of different physiological parameters have to be determined, it is necessary to use different questionnaires as generally each questionnaire is dedicated to a single parameter.

Systems for determining values of physiological parameters may typically focus on a single parameter, such as gaze direction or glare situation.

Systems for determining gaze direction may be either non-portable or energy intensive.

Systems for detecting glare situation may use the amount of environméntal light and detect a possible glare situation based on this amount. However, this amount may not be a parameter directly related to the glare situation of the individual.

There is a need for systems, head-mountable devices and methods for determining a value of a physiological parameter of an individual.

### Summary

The following presents a simplified summary to provide a basic understanding of various aspects of this disclosure. This summary is not an extensive overview of all contemplated aspects and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a system for determining a value of a physiological parameter of an individual. The system comprises a head-mountable device intended to be worn by the individual. The head-mountable device comprises a first set of at least one sensor and a second set of at least one sensor. The at least one sensor of the first set comprises at least one LED and at least one photodiode. The at least one LED is arranged, so that light emitted by the at least one LED is reflected by the face of the individual and received by the at least one photodiode. Values outputted by the at least one sensor of the first set depend on an amount of the light received by the at least one photodiode. Values outputted by the at least one sensor of the second set depend on a distance between the face of the individual and the head-mountable device. The system is configured to determine the value of the physiological parameter of the individual based on values outputted by the at least one sensor of the first set and the values outputted by the at least one sensor of the second set.

Another aspect of this disclosure is a head-mountable device for determining a value of a physiological parameter of an individual. The head-mountable device comprises a first set of at least one sensor and a second set of at least one sensor. The at least one sensor of the first set comprises at least one LED and at least one photodiode. The at least one LED is arranged, so that light emitted by the at least one LED is reflected by the face of the individual and received by the at least one photodiode. Values outputted by the at least one sensor of the first set depends on an amount of the light received by the at least one photodiode. Values outputted by the at least one sensor of the second set depend on a distance between the face of the individual and the head-mountable device. The head-mountable device is configured to output a value of a physiological parameter of the individual based on values outputted by the at least one sensor of the first set and the values outputted the at least one sensor of the second set.

Another aspect of this disclosure is a method for determining a value of a physiological parameter of an individual. The method comprises receiving values from at least one sensor of a first set of sensors of the head-mountable device and receiving values from at least one sensor of a second set of sensors of the head-mountable device. The at least one sensor of the first set comprises at least one LED and at least one photodiode. The at least one LED is arranged, so that light emitted by the at least one LED is reflected by the face of the individual and received by the at least one photodiode. The values received from the at least one sensor of the first set depend on an amount of the light received by the at least one photodiode. The values received from the at least one sensor of the second set depend on a distance between the face of the individual and the head-mountable device. The method also comprises determining the value of a physiological parameter of the individual based on the values received from the at least one sensor of the first set and the values received from the at least one sensor of the second set.

Another aspect of this disclosure is a computer program comprising instructions which, when the computer program is executed by a computer, cause the computer to carry out a method for determining a value of a physiological parameter of an individual. The method comprises receiving values from at least one sensor of a first set of sensors of the head-mountable device and receiving values from at least one sensor of a second set of sensors of the head-mountable device. The at least one sensor of the first set comprises at least one LED and at least one photodiode. The at least one LED is arranged, so that light emitted by the at least one LED is reflected by the face of the individual and received by the at least one photodiode. The values received from the at least one sensor of the first set depend on an amount of the light received by the at least one photodiode. The values received from the at least one sensor of the second set depend on a distance between the face of the individual and the head-mountable device. The method also comprises determining the value of a physiological parameter of the individual based on the values received from the at least one sensor of the first set and the values received from the at least one sensor of the second set.

Another aspect of this disclosure is a non-transitory program storage device, readable by a computer, tangibly embodying a program of instructions executable by the computer to perform a method for determining a value of a physiological parameter of an individual. The method comprises receiving values from at least one sensor of a first set of sensors of the head-mountable device and receiving values from at least one sensor of a second set of sensors of the head-mountable device. The at least one sensor of the first set comprises at least one LED and at least one photodiode. The at least one LED is arranged, so that light emitted by the at least one LED is reflected by the face of the individual and received by the at least one photodiode. The values received from the at least one sensor of the first set depend on an amount of the light received by the at least one photodiode. The values received from the at least one sensor of the second set depend on a distance between the face of the individual and the head-mountable device. The method also comprises determining the value of a physiological parameter of the individual based on the values received from the at least one sensor of the first set and the values received from the at least one sensor of the second set.

### Description of the drawings

For a more complete understanding of the description provided herein and the advantages thereof, reference is now made to the brief descriptions below, taken in connection with the accompanying drawings and detailed description, wherein like reference numerals represent like parts.
Figure 1 represents a system for determining a value of a physiological parameter of an individual.
Figure 2 represents eyeglasses.
Figure 3 represents another example of the eyeglasses.
Figure 4 represents another example of the eyeglasses.
Figure 5 represents the method for determining a value of a physiological parameter of an individual.
Figures 6-a and 6-b represent an example of use of a head-mountable device.

### Detailed description of embodiments

The detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form to avoid obscuring such concepts.

### Description of the system for determining a value of a physiological parameter of an individual

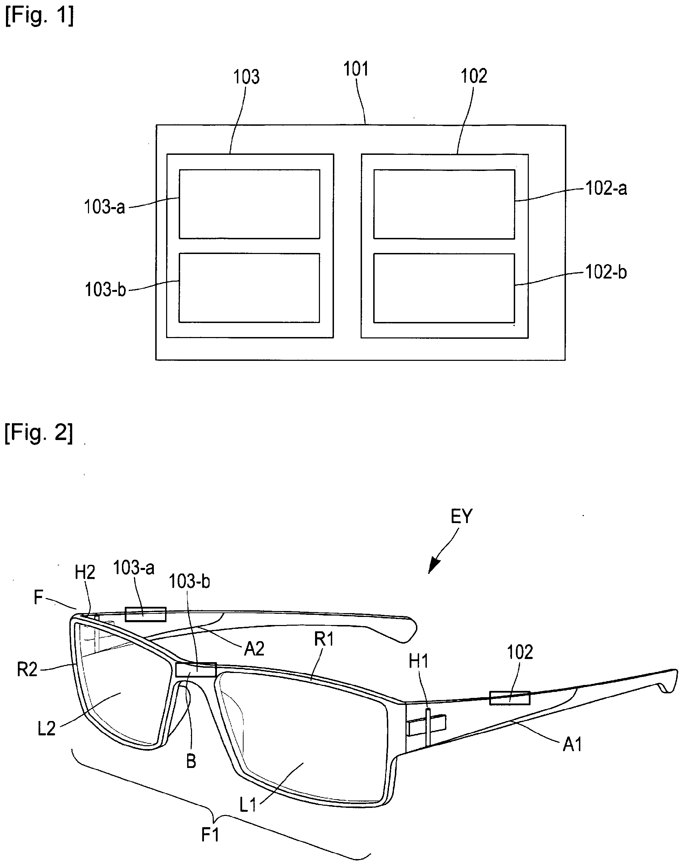

Figure 1 represents a system 101 for determining a value of a physiological parameter of an individual. The system 101 may comprise a calculation module 102 and a head-mountable device 103. The head-mountable device 103 may be intended to be worn by the individual.

The calculation module 102 may be configured for determining the value of the physiological parameter of the individual.

The head-mountable device 103 may comprise a first set 103-a of at least one sensor and a second set 103-b of at least one sensor.

The at least one sensor of the first set 103-a may comprise at least one LED and at least one photodiode. The at least one LED may be arranged, so that light emitted by the at least one LED may be reflected by the face of the individual and may be received by the at least one photodiode. To be arranged so that so that light emitted by the at least one LED may be reflected by the face of the individual and may be received by the at least one photodiode, the at least one LED may face a face of the individual.

Values outputted by the at least one sensor of the first set 103-a may depend on an amount of the light received by the at least one photodiode.

Values outputted by the at least one sensor of the second set 103-b may depend on a distance between the face of the individual and the head-mountable device 103.

More precisely the values outputted by the at least one sensor of the second set 103-b may varied with a slippage level of the head-mountable device 103 on the nose, in a monotonic, and generally stable over time, manner.

More precisely the values outputted by the at least one sensor of the second set 103-b may have a bijective relationship with the slippage level.

The at least one sensor of the second set 103-b may comprise at least one LED and at least one photodiode. Advantageously the at least one LED and at least one photodiode may be arranged so that the light emitted by the at least one LED is reflected by a part of the head of the wearer of the head-mountable device 103 at the junction area between the bridge of the nose and the forehead.

The at least one sensor of the second set 103-b may be a time-of-flight sensor, a LIDAR or proximity sensor.

To have values outputted by the at least one sensor of the second set 103-b depending on a distance between the face of the individual and the head-mountable device 103, the at least one photodiode and at the at least one LED of the second set 103-b may be located at less than 1. cm, for example less than 5 mm, preferably less than 2 mm of the face of the individual.

The LEDs may be IR LEDs and the photodiodes may be active IR photodiodes allowing to be more robust to light changes of the external environment. More precisely, using IR and modulated light allows avoiding any perturbations from visible light and the modulation of the emitted light allows the photodiode to differentiate the light emitted by the LED of environment lights.

The system 101, more precisely the calculation module 102, may be configured to determine the value of the physiological parameter of the individual based on values outputted by the at least one sensor of the first set 103-a and the values outputted by the at least one sensor of the second set 103-b.

By "head-mountable device", one means a device designed to be mounted on the head of the individual. The head-mountable device 103 may perform several functions, some of which are related to the eyes of the individual.

Examples of head-mountable devices include eyewear, helmets, and clips that can be attached to a cap or eyewear, or a mask.

Eyewear encompasses any type of head mountable devices designed to be worn in or in front at least one eye of a wearer, in any suitable form factor.

Eyewear includes head-mounted display devices (e.g. near eye display devices, augmented reality devices, virtual reality devices or mixed reality devices), helmets (with a visor), eyeglasses, googles, masks, visors, contact lenses, intraocular implants.

The head-mountable device 103 may comprise an active optical element.

By an active optical element, one means an optical element in which a value of a parameter of a functionality of the optical element may be modified. The value of the parameter may be modified by applying a signal, for example an electrical signal to terminals of the active optical element. The functionality may be one of the following:
- a transmittance level of the active optical element,
- a tint of the active optical element,
- a reflectance level of the active optical element and
- a parameter of a polarization of the active optical element, for example a polarization angle of the active optical element,
- an optical power of the active optical element,
- elements displayed by the active optical element. The elements may be images, symbols, letters, or texts. In embodiments, the elements to be displayed and the position of these elements may be selected.

The figure 2 represents eyeglasses EY, which are an example of the head-mountable device 103. The eyeglasses EY comprise two lenses L1 and L2 and a frame F. The frame F comprises two arms or temples A1 and A2 and a front part F1. The front part F1 comprises a right rim R1 and left rim R2 linked together by a bridge B. The front part F1 and the two arms A1 and A2 are linked using two hinges H1 and H2. The hinges H1 and H2 allow the individual to fold the arms A1 and A2 along the front part F1. The rims R1 and R2 of the frame F1 are configured to receive and to maintain the lenses L1 and L2. The eyeglasses EY may be prescription eyeglasses for which the refraction has been adapted to the individual by an eye care professional or eyeglasses without refraction for example sunglasses.

The distance between the face of the individual and the eyeglasses EY may correspond to a slippage of the eyeglasses EY.

Figure 2 also represents an example of a localisation of the first set 103-a of the at least one sensor and the second set 103-b of the at least one sensor. In this example the at least one sensor, for example the at least one photodiode and the at least one LED, of the second set 103-b are located in a bridge B of the eyeglasses EY.

The at least one sensor of the second set 103-b may also be located in other positions like on one of the two arms A1 or A2. Preferably the at least one sensor of the second set 103-b may be oriented to a zone where no movement are expected (for example cheek, nose, temples, forehead) meaning that if a change in signal is detected it may be with a high certainty the frame slippage.

The position of the head-mountable device 103, and more precisely of the frame F for the eyeglasses EY, over the head may be a meaningful insight because it impacts the spatial position of the at least one sensor of the first set 103-a around the eye. Depending on the slipping of the head-mountable device 103, the signals coming from the at least one sensor of the first set 103-a may not be the same.

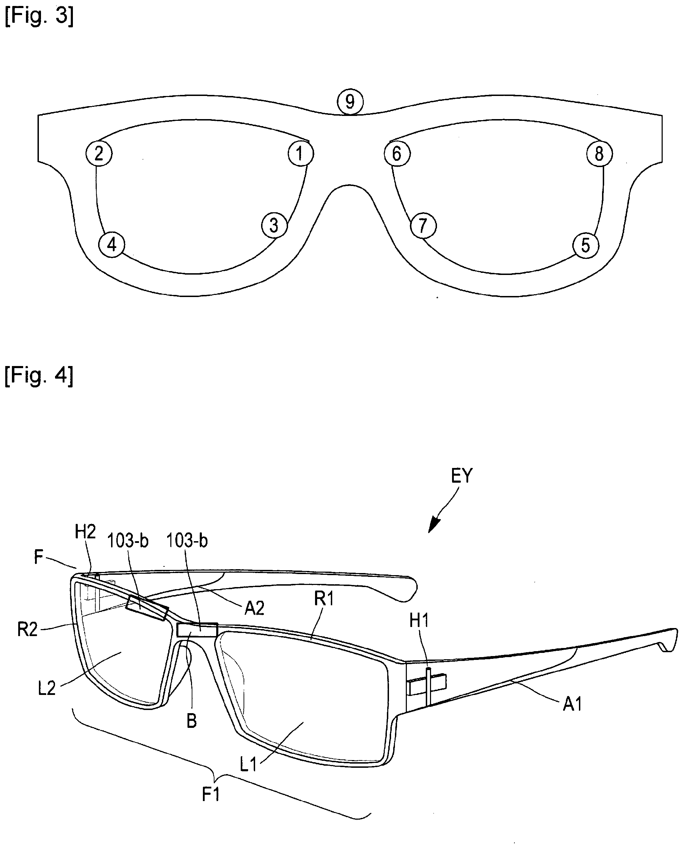

The figure 3 represents another arrangement of the sensors. In this arrangement, the sensors 1 to 8 are sensors of the first set 103-a and the sensor 9 is the sensor of the second set 103-b.

The calculation module 102 may comprise a memory 102-a and a processor 102-b.

The calculation module may be a low resource calculation module.

Examples of processors 102-b include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, NPU (Neural Processing Unit) and other suitable hardware configured to perform the various functionality described throughout this disclosure.

The memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor 102-a of the calculation module 102.

The head-mountable device 103 may comprise the calculation module 102.

When the head-mountable device 103 is the eyeglasses EY, the calculation module 102, as represented in the figure 2, may be located in one or both of the arms A1 and A2 or in the front face F1.

In other embodiments the calculation module 102 may be integrated in a smartphone or a watch more precisely a wristwatch.

Wristwatches are designed to be worn around the wrist, attached by a watch strap or other type of bracelet, including metal bands, leather straps, or any other kind of bracelet.

The watch may also be a smartwatch that is a wearable computer in the form of a watch. The calculation module 102 may be included in the smartwatch and may be share with other functions of the smartwatch.

The calculation module 102 may be a computer or may be a virtual machine located on a cloud network or a server not co-located with the individual.

The head-mountable device 103 may also comprise an eye tracker.

The figure 4 represents another possible localisation of the at least one sensor of the second set 103-b, more precisely when the at least one sensor of the second set 103-b is a proximity sensor.

### Description of the method for determining a value of a physiological parameter of an individual

To determine a value of a physiological parameter of an individual, the memory 102-a may store a computer program comprising instructions which, when the program is executed by the processor 102-b, cause the calculation module 102 to carry out a method, for example a computer implemented method, for determining a value of a physiological parameter of an individual.

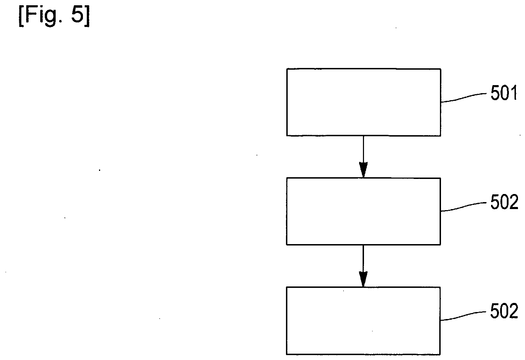

The method, as presented in figure 5, comprises:
- a step 501 of receiving values from the at least one sensor of the first set 103-a and
- a step 502 of receiving values from the at least one sensor of the second set 103-b,
- a step 503 of determining the value of a physiological parameter of the individual based on the values received from the at least one sensor of the first set 103-a and the values received from the at least one sensor of the second set 103-b.

The physiological parameter may be chosen among:
- gaze direction of the individual,
- ocular movement of the individual,
- eye fatigue of the individual and
- glare situation of the individual.

Depending on the value of the physiological parameter, one may modify the value of the parameter of the functionality of the active optical element.

Gaze direction of the individual: This reflects the orientation or line of sight of the eyes of the individual, which is a physiological behaviour related to visual focus and attention.

Ocular movement of the individual: This refers to the motion of the eyes, such as saccades, fixations, blinks, or vergence movements, which are physiological processes governed by ocular muscles and neural control.

Eye fatigue of the individual: This is a physiological state that may result from prolonged visual activity, associated with muscle strain, discomfort, or reduced visual efficiency. The eye fatigue may also result from prolonged use of screens, and the symptoms are very broad: dry eyes, redness, a gritty sensation in the eyes, and stinging. The eye fatigue may also affect the muscles in the peri-orbital area.

Glare situation of the individual: While "glare situation" may depend on external environmental factors, its impact on the individual involves physiological responses like pupil constriction, blinking, or discomfort due to excessive light. Glare may also affect the muscles in the peri-orbital area (e.g., frowning, widening of the eyes) and may also result from disturbing point light sources.

In embodiments the method of figure 5 may comprise a step of inputting the values received from the at least one sensor of the first set 103-a and the values received from the at least one sensor of the second set 103-b in a machine learning model dedicated to the determination of the value of the physiological parameter.

The values may be raw values. By raw values one means values directly extracted from the signal outputted by the at least one sensor of the first set 103-a and the at least one sensor of the second set 103-b.

The values may be features extracted from a time window of the signal (mean of the signal, maximum of the signal, minimum of the signal, variance of the signal, number of peaks in the signal, amplitude of these peaks, coefficients of Fourier transform of the signal ...).

The machine learning model dedicated to the determination of the value of the physiological parameter may be previously trained with:
- model values from the at least one sensor of the first set 103-a,
- model values from the at least one sensor of the second set 103-b,
- model values of the physiological parameter associated with the model values from the at least one sensor of the first set 103-a and the model values from the at least one sensor of the first set 103-b.

The model values may be raw values. By raw values one means values directly extracted from the signal outputted by the at least one sensor of the first set 103-a and the at least one sensor of the second set 103-b.

The model values may be features extracted from a time window of the signal (mean of the signal, maximum of the signal, minimum of the signal, variance of the signal, number of peaks in the signal, amplitude of these peaks, coefficients of Fourier transform of the signal ...).

The model values may be experimental data coming from a plurality of model individuals. This is relevant if one wants to have only one model for all individuals. It shows how well the model may generalize to different individuals. This also has the advantage of not having to re-train the model and avoiding the requirement of calibration when the system 101 is used by a new individual. It also has the advantage of being easier to implement.

The model values may be experimental data coming from the individual. This is relevant in the case where one wants the model to be tailored to a specific individual. It shows how well the model may generalize to different recordings with different positions of the head-mountable device 103, while keeping a general similarity between the recordings.

The model values may be for different positions of the head-mountable device 103, more precisely for different slippage of the head-mountable device 103.

The model value may also be taken for a variety of model individual. The model value may also be taken for a variety of different head-mountable device 103. These allow the collection of data in different condition (morphology, frame position, eye movement) allowing leverage the ability to discriminate all possible values of the physiological parameter.

The model data may be obtained during a process in which the model individuals are required to perform a series of tasks in various positions of the head-mountable device 103. These tasks include natural eye movements, saccade movements, fixation movements, pursuit movements and fixation.

For the natural eye movement, the model individuals may be presented with an image in which they are required to find different elements. To do this the model individuals may have to examine the whole image.

For the saccadic and fixation movements, a video of points may be shown to the model individuals. Model individuals may be instructed to fixate one of the points, with each point appearing for 90 milliseconds. The appearance of these points may be randomized.

For pursuit movements, model individuals may be instructed to track a moving point that moves vertically then horizontally.

During the collecting of model data, one may induce visual fatigue in the model individuals by using specific stimuli. For example, one may use the specific task developed by Pr Rosenfielfd and presented in the article Rosenfield, M., Hue, J. E., Huang, R. R., & Bababekova, Y. (2012). The effects of induced oblique astigmatism on symptoms and reading performance while viewing a computer screen. The modifications of the values outputted by the at least one sensor of the first set 103-a and the at least one sensor of the second set 103-b represent optical variations of reflectance in the eye area, related to physiological variation in this region. For example, they may represent variation of pupil size, eye movements, muscles. To determine the visual fatigue one may use a subjective questionnaire related to the visual fatigue of the model individual.

In embodiments based on the values, outputted by the at least one sensor of the second set 103-b, one may select a machine learning model, or may change the values of the parameters or hyperparameters of the machine learning model. For example, one may choose different neural networks.

During the training, the model values from the at least one sensor of the first set 103-a and the model values from the at least one sensor of the first set 103-b are inputted to the machine learning model and the model values of the physiological parameter are targets with which the outputs of the machine learning model are compared. Based on the differences between the outputs of the machine learning and the model values of the physiological parameter, the values of the parameters of the machine learning model are updated.

The machine learning model dedicated to the determination of the value of the physiological parameter may be chosen among:
- a convolutional neural network,
- a recurrent neural network,
- a transformer
- a decision tree,
- a random forest,
- support vector machine and
- a linear model.

Preferably when using the convolutional neural network, the recurrent neural network, or the transformer, one may use directly the raw values extracted from the signal outputted by the at least one sensor of the first set 103-a and the at least one sensor of the second set 103-b.

Preferably when using a decision tree, a random forests classifier, support vector machines or a linear model, one may use directly the features extracted from a time window of the signal outputted by the at least one sensor of the first set 103-a and the at least one sensor of the second set 103-b.

When the physiological parameter is the gaze direction of the wearer, as the current gaze direction generally does not highly depend on previous gaze directions, the decision tree, the random forest the support vector machine and the linear model may be used with the raw values extracted from the signal outputted by the at least one sensor of the first set 103-a and the at least one sensor of the second set 103-b.

Depending on the physiological parameter, the machine learning model may a classification or a regression and the outputs may be respectively discrete or continuous.

The support vector machine may be used with a gaussian kernel and be trained by using a L2 penalty = 0.01 and by giving more weight on underrepresented class.

The at least one decision tree may be trained using gradient boosting for example implemented using the open-source software Light GBM. The values of the hyperparameters may be: number of leaves = 9, L1 penalization coefficient = 0.01. The penalization coefficient may be used during the training, it is not a hyperparameter of the model itself. The following features from the signal may be used with the decision tree:
- Average values of sensors over a selected time window,
- Standard deviation values of sensors over a selected time window,
- Estimation of 1^{st} derivative,
- Estimation of 2^{nd} derivative.
When the physiological parameter is the gaze direction of the wearer, the raw values extracted from the signal outputted by the at least one sensor of the first set 103-a and the at least one sensor of the second set 103-b.

The random forests classifier may be a balanced random forests classifier with the hyperparameters having the following values: minimum number of samples required to be at a leaf node = 24. The following adaptation parameters may be used with the balanced random forests classifier:
- Average values of sensors over a selected time window,
- Median values of sensors over a selected time window,
- Maximum values of sensors over a selected time window,
- Minimum values of sensors over a selected time window,
- Standard deviation values of sensors over a selected time window.

In an embodiment the transformer may be a time series transformer (TST) with the hyperparameters having the following values: number of attention head = 8, number of sub-encoder layers = 3. Raw values of each sensor may be used with the Transformer.

In an embodiment the convolutional neural network may be composed of 3 convolutional blocks comprising 128 kernels of size 5 or 7, a batch normalization operation and rectified linear unit function (ReLU). A classification block comprising a linear transformation with 128 neurons and a ReLU function could follow the convolutional block.

In an embodiment the recurrent neural network may be composed of a long-short term memory (LSTM) block having 3 recurrent layers with hidden state of dimension 64.

In embodiments the head-mountable device 103 may be configured for determining its position over the head of the individual. The head-mountable device 103 may also be configured to determine if it is worn or not. This may be done by using only the signals outputted by the at least one sensor of the second set 103-b. This may also be done by using the signal outputted by the at least one sensor the first set 103-a and the at least one sensor the second set 103-b.

The determined position over the head may be considered by the eye tracker of the head-mountable device 103. This will allow a better determination of the gaze direction.

The determined position over the head may be considered by the active optical element of the head-mountable device 103. This will allow a modification of the way the active optical element is activated, for example by moving the area in which elements are displayed.

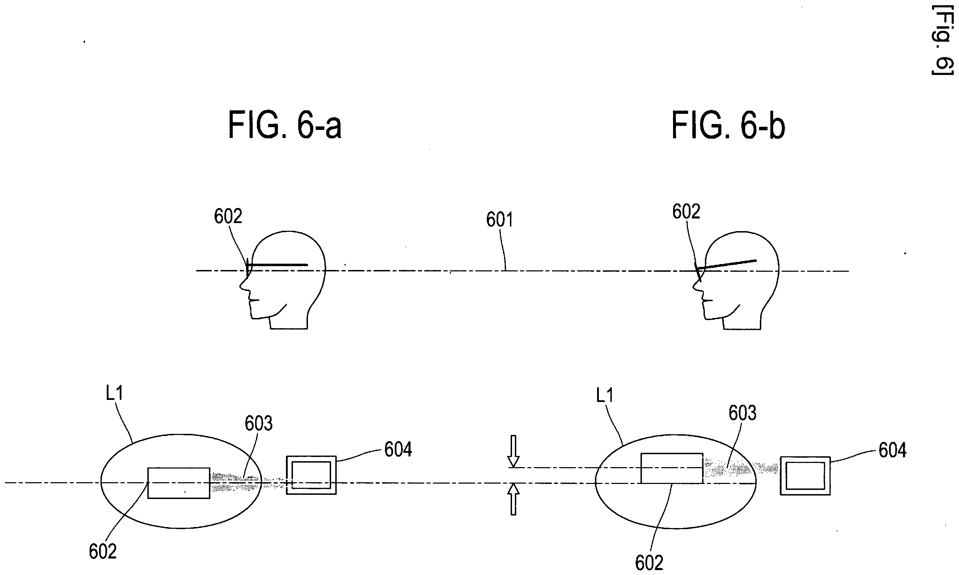

The figures 6-a and 6-b represent how the area in which elements are displayed may be moved according to the determined position of the head-mountable device 103 over the head.

Figure 6-a represents a regular fit of the eyeglasses EY. In this situation the light of sight 601 is parallel to the arms A1 and A2 of the eyeglasses EY. In this case an ideal localisation of an area 602 in which the elements are displayed by the active optical element is in the middle of the lens L1.

Figure 6-b represents a slipped fit of the eyeglasses EY. In this situation the light of sight 601 is also parallel to the arms A1 and A2 of the eyeglasses EY. In this case the ideal localisation of the area 602 in which the elements are displayed by the active optical element is in the top of the lens L1.

## Claims

1. System (101) for determining a value of a physiological parameter of an individual, the system (101) comprising a head-mountable device (103) intended to be worn by the individual, the head-mountable device (103) comprising:
- a first set (103-a) of at least one sensor and
- a second set (103-b) of at least one sensor
the at least one sensor of the first set (201) comprising at least one LED and at least one photodiode, the at least one LED being arranged, so that light emitted by the at least one LED is reflected by the face of the individual and received by the at least one photodiode,
values outputted by the at least one sensor of the first set (103-a) depend on an amount of the light received by the at least one photodiode,
values outputted by the at least one sensor of the second set (103-b) depend on a distance between the face of the individual and the head-mountable device (103),
the system (101) being configured to determine the value of the physiological parameter of the individual based on values outputted by the at least one sensor of the first set (103-a) and the values outputted by the at least one sensor of the second set (103-b).

2. The system (101) according to the claim 1, the system also comprising a calculation module (102), the calculation (102) module being configured to determine the value of the physiological parameter of the individual based on the values outputted by the at least one sensor of the first set (103-a) and the values outputted by the at least one sensor of the second set (103-b).

3. The system (101) according to the claim 1 or 2,
the at least one sensor of the second set (103-b) comprising at least one LED and at least one photodiode.

4. The system (101) according to the claim 3,
the at least one LED and the at least one photodiode of the second set (202) being located so that the reflected light received by the at least one photodiode of the second set (103-b) predominantly depends on the distance between the face of the individual and the head-mountable device and depends minimally on the value of the physiological parameter.

5. The system (101) according to the claim 3 or 4,
the at least one photodiode and at the at least one LED of the second set (103-b) being located at less than 1 cm, for example less than 5 mm, preferably less than 2 mm of the face of the individual.

6. The system (101) according to the claim 3 or 4,
the at least one photodiode and the at least one LED of the second set (103-b) being located in a bridge of a frame of the head-mountable device (103).

7. The system (101) according to any one of the claims 1 to 6,
the at least one sensor of the second set (103-b) being a time-of-flight sensor or a LIDAR.

8. The system (101) according to any one of the claims 1 to 7,
the head-mountable device (103) being eyewear.

9. The system (101) according to any one of the claims 1 to 8,
the system comprising a machine learning model previously trained using model values outputted by the at least one sensor of the first set, model values outputted by the at least one sensor of the second set and model values of the physiological parameter of model individuals.

10. The system (101) according to the claim 9,
the machine learning model being chosen among:
- a convolutional neural network,
- a decision tree,
- a random forests classifier,
- support vector machines and
- logistic regression.

11. The system (101) according to any one of the claims 1 to 10,
the physiological parameter being chosen among:
- gaze direction of the individual,
- ocular movement of the.individual,
- eye fatigue of the individual and
- glare situation of the individual.

12. Head-mountable device (103) comprising:
- a first set (103-a) of at least one sensor and
- a second set (103-b) of at least one sensor
the at least one sensor of the first set (103-a) comprising at least one LED and at least one photodiode, the at least one LED being arranged, so that light emitted by the at least one LED is reflected by the face of the individual and received by the at least one photodiode,
values outputted by the at least one sensor of the first set (103-a) depend on an amount of the light received by the at least one photodiode,
values outputted by the at least one sensor of the second set (103-b) depend on a distance between the face of the individual and the head-mountable device (103),
the head-mountable device (103) being configured to output a value of a physiological parameter of the individual based on values outputted by the at least one sensor of the first set (103-a) and the values outputted the at least one sensor of the second set (103-b).

13. The head-mountable device (103) according to the claim 12,
the at least one sensor of the second set (103-b) comprising at least one LED and at least one photodiode.

14. The head-mountable device (103) according to the claim 13,
the at least one LED of the second set (103-b) and the at least one photodiode of the second set (103-b) being located so that the reflected light received by the at least one photodiode of the second set (103-b) predominantly depends on the distance between the face of the individual and the head-mountable device (103) and depends minimally on the value of the physiological parameter.

15. Computer implemented method for determining a value of a physiological parameter of an individual wearing a head-mountable device (103),
the method comprising:
- receiving (501) values from at least one sensor of a first set (103-a) of sensors of the head-mountable device (103) and
- receiving (502) values from at least one sensor of a second set (103-b) of sensors of the head-mountable device (103),
the at least one sensor of the first set (103-a) comprising at least one LED and at least one photodiode, the at least one LED being arranged, so that light emitted by the at least one LED is reflected by the face of the individual and received by the at least one photodiode,
the values received from the at least one sensor of the first set (103-a) depend on an amount of the light received by the at least one photodiode,
the values received from the at least one sensor of the second set (103-b) depend on a distance between the face of the individual and the head-mountable device (103),
the method also comprising:
- determining (503) the value of a physiological parameter of the individual based on the values received from the at least one sensor of the first set (103-a) and the values received from the at least one sensor of the second set (103-b).
